(19) Europäisches Patentamt
European Patent Office
Office européen des brevets

(11) Numéro de publication : **0 522 937 A1**

(12) # DEMANDE DE BREVET EUROPEEN

(21) Numéro de dépôt : **92401903.7**

(22) Date de dépôt : **03.07.92**

(51) Int. Cl.[5] : **A61K 47/10**, A61K 47/26, A61K 47/44

(30) Priorité : **08.07.91 FR 9108527**

(43) Date de publication de la demande : **13.01.93 Bulletin 93/02**

(84) Etats contractants désignés : **PT**

(71) Demandeur : **RHONE-POULENC RORER SA
20, avenue Raymond Aron
F-92160 Antony (FR)**

(72) Inventeur : **Bastard, Jean-Pierre
12 rue des Bergères
F-77150 Lesigny (FR)**
Inventeur : **Dupechez, Thierry
2 Avenue Paul Doumer
F-92360 Villemoisson sur Orge (FR)**
Inventeur : **Fabre, Jean-Louis
9 rue Fagon
F-75013 Paris (FR)**

(74) Mandataire : **Le Pennec, Magali et al
RHONE-POULENC RORER SA, Direction des Brevets, 20 Avenue Raymond Aron
F-92165 Antony Cédex (FR)**

(54) **Compositions à base de dérivés de la classe des taxanes.**

(57) La présente invention concerne de nouvelles compositions à base de dérivés de la classe des taxanes constituées de solutions de ces dérivés dans un mélange de solvant composé d'éthanol et de polysorbate.

Ces solutions sont utilisées pour préparer des perfusions.

EP 0 522 937 A1

Jouve, 18, rue Saint-Denis, 75001 PARIS

La présente invention concerne une nouvelle forme pharmaceutique à base d'un agent thérapeutique ayant une activité antitumorale et antileucémique. Elle concerne plus particulièrement une nouvelle forme injectable contenant des produits de la famille des taxanes tels que notamment le taxol ou un de ses analogues ou dérivés de formule générale suivante:

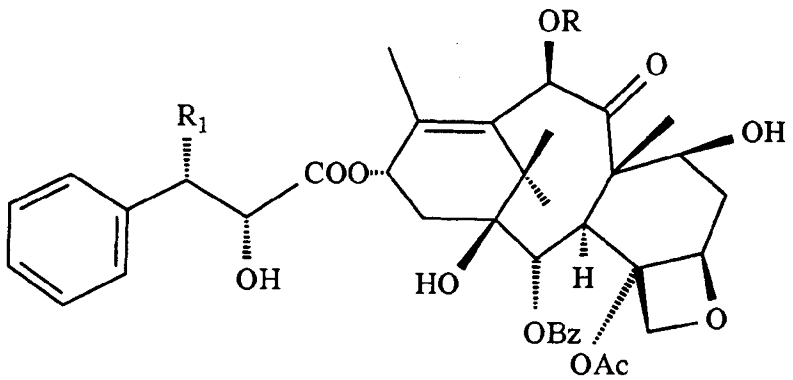

(I)

Dans la formule (I), R représente un atome d'hydrogène ou un radical acétyle, le symbole $R_1$ radical tertiobutoxycarbonylamino ou benzoyloxyamino. On préfère parmi l'ensemble de ces dérivés les deux dérivés pour lesquels R représente un groupe acétyle et $R_1$ un groupe benzoyloxyamino ou celui pour lequel R représente un atome d'hydrogène et $R_1$ un radical tertio butoxycarbonylamino.

Le premier de ces deux composés est plus connu sous la dénomination de taxol, le deuxième est connu sous la dénomination de Taxotère.

Ces produits présentent in vivo une activité importante sur les tumeurs malignes ce qui a permis de les étudier dans le traitement des maladies résistantes à toutes les autres thérapies anticancéreuses.

Malheureusement ces produits présentent une solubilité dans l'eau tellement faible qu'il a été nécessaire de préparer une formulation pour préparation injectable à base d'agent tensioactif et d'éthanol. L'éthanol, est le meilleur solvant qui permette de solubiliser les molécules répondant à la formule (I).

A titre d'exemple, selon la publication de Rowinsky, Lorraine, Cazenave et Donehower parue dans le Journal of the National Cancer Institute, vol. 82, No 15, pages 1247 à 1259, le 1er Août 1990, on prépare une première solution, dite "solution mère", contenant environ 6 mg/ml de taxol dans un mélange solvant composé de:

- 50 % en volume d'éthanol
- 50 % en volume de Crémophor EL.

Lors de l'injection, cette solution est mélangée avec un liquide de perfusion contenant du chlorure de sodium ou du dextrose. Pour obtenir un mélange stable, d'un point de vue physique comme d'un point chimique, les auteurs de cet article disent qu'il faut limiter la concentration en principe actif dans le soluté de perfusion à des concentrations d'environ 0,03 à 0,6 mg/ml (voir publication précédente page 1251 colonne 1, troisième paragraphe).

Or il est souhaitable de pouvoir injecter des doses suffisantes de principe actif, pour cela les cliniciens désirent injecter des concentrations en principe actif comprises entre environ 0,3 et 1 mg/ml dans le liquide de perfusion, au delà de ces doses apparaissent des phénomènes de chocs anaphylactiques difficiles à maîtriser dus pour l'essentiel au Cremophor (voir la publication de Rowinsky page 1250 deuxième colonne dernier paragraphe).

Toujours selon cette publication, pour obtenir de telles concentrations (entre 0,3 et 1mg/ml) il est nécessaire d'injecter des solutions contenant en même temps que le principe actif des concentrations en chacun des composés suivants, éthanol et surtout Crémophor, d'environ 8 g pour 100 ml de soluté. Le traitement demandant souvent l'administration de doses élevées de principe actif et la concentration du principe actif dans la solution étant relativement faible l'injection de fort volume a pour effet de provoquer durant le traitement en plus des manifestations anaphylactiques des manifestations d'éthylisme.

Il a été découvert par la mise en oeuvre des formes pharmaceutiques de la présente invention que l'on pouvait supprimer le Crémophor et diminuer fortement les concentrations en éthanol.

Pour cela on prépare une solution mère contenant le principe actif dans un mélange de solvants composé d'éthanol qui est le meilleur solvant biocompatible des principes actifs de la classe des taxanes et d'un agent tensioactif choisi parmi les polysorbates commercialisés notamment sous la dénomination Tween.

La solution mère est préparée par dissolution du principe actif dans l'éthanol puis addition progressive de

l'agent tensioactif. On peut ainsi préparer des solutions contenant 10 à 100 mg/ml de principe actif dans un mélange contenant environ 50 % d'agent tensioactif.

L'objet de la présente invention est le suivant: le Crémophor décrit dans la publication du journal of National Cancer Institute a été remplacé par un polysorbate. En effet, lorsque l'on utilise un soluté injectable contenant de l'éthanol et comme agent tensioactif le polysorbate 80 à la place du Crémophor, au niveau clinique, il est apparu que les réactions anaphylactiques étaient fortement diminuées par rapport à l'utilisation du même soluté préparé avec le Crémophor. En plus de cet avantage considérable, il est apparu de façon tout à fait étonnante que dans les flacons de solution mère, la concentration en principe actif peut atteindre 15 mg/ml. Le liquide de perfusion après dilution de ces flacons contient une quantité d'éthanol comme une quantité de tensioactif diminuée par un peu plus de deux.

Les perfusions préparées à partir des solutions mères précédentes et contenant une concentration en principe actif de, par exemple, 1 mg/ml, ce qui est une préférence, contiennent moins de 50 ml/l d'agent tensioactif et d'éthanol, ce qui représente une diminution d'environ 40 % par rapport aux perfusions de l'art antérieur.

Ces perfusions sont stables d'un point de vue physique, c'est à dire qu'on ne voit apparaître aucun phénomène de précipitation avant environ 8 heures.

Les perfusions de taxol ou de Taxotère sont ensuite injectées à l'homme à un débit prédéterminé en fonction de la quantité de principe actif que l'on veut injecter. On n'observe pas avec ces solutions les phénomènes de chocs anaphylactiques que l'on observait avec les solutions de l'art antérieur.

L'invention sera plus complètement décrite à l'aide des exemples suivants qui ne doivent pas être considérés comme limitatifs de l'invention.

EXEMPLES SELON L'INVENTION

**EXEMPLE 1**

On dissout 0,450 g de Taxotère dans 15 ml d'éthanol. On complète avec le polysorbate 80 jusqu'à 30 ml pour obtenir une solution contenant 15 mg/ml de Taxotère. La stabilité physicochimique de cette solution est satisfaisante.

Cette solution après mélange avec une solution de glucose à 5 % de façon à obtenir une concentration finale de 1 mg/ml contient environ 33 ml/l de polysorbate 80 et 33 ml/l d'éthanol.

La perfusion est stable pendant plus de 21 heures. On entend par stabilité physique le fait de ne voir aucun phénomène de précipitation pendant cette période.

**EXEMPLE 2**

On reproduit le même exemple avec une concentration initiale de 10 mg/ml de Taxotère les résultats sont indiqués dans le tableau 1.

EXEMPLE COMPARATIF SELON L'ART ANTERIEUR

On dissout 0,180 g de taxol dans 15 ml d'éthanol. On complète avec du Crémophor pour obtenir 30 ml d'une solution qui contient 6 mg/ml de taxol.

Cette solution est diluée dans le même soluté de perfusion que précédemment à raison de 1 mg/ml, le soluté de perfusion contient 87,7 ml/l de Cremophor et 87,7 ml/l d'éthanol.

La solution de perfusion est stable pendant plus de 21 heures.

**EXEMPLE SELON L'INVENTION AVEC LE TAXOL**

On reproduit le même exemple que l'exemple selon l'art antérieur en remplaçant le cremophor par le polysorbate 80. Les résultats de stabilité sont indiqués dans le tableau1.

**EXEMPLE 3**

On dissout 65 g de Taxotère dans 2083 ml d'éthanol. On ajuste à 4147 ml par addition de 2083 ml de polysorbate 80. On homogénéise par agitation mécanique. On filtre sur un filtre de dimension de pores 0,2 µm. On obtient une solution à environ 15 mg/ml de Taxotère.

Cette solution après dilution à une teneur en Taxotère de 1 mg/ml dans une poche de perfusion contenant du dentrose à 5 % est stable au moins 96 heures.

TABLEAU 1 SELON LE PREMIER OBJET DE L'INVENTION

| produit | Solvant | Solution mère concentration | principe actif dans la perfusion | Tensioactif dans la perfusion | Ethanol dans la perfusion | Stabilité |
|---|---|---|---|---|---|---|
| taxol | EtOH/Crem | 6 mg/ml | 1 mg/ml | 87,7 ml/l | 87,7 ml/l | >21H |
| taxol | EtOH/Poly | 6 mg/ml | 1 mg/ml | 83,3 ml/l | 83,3ml/l | >21H |
| Taxotère | EtOH/Poly | 15 mg/ml | 1 mg/ml | 33,3 ml/l | 33,3 ml/l | >21H |
| Taxotère | EtOH/Poly | 10 mg/ml | 1 mg/ml | 50 ml/l | 50 ml/l | >21H |

**Revendications**

**-1-** Nouvelles compositions à base d'un dérivé de formule (I)

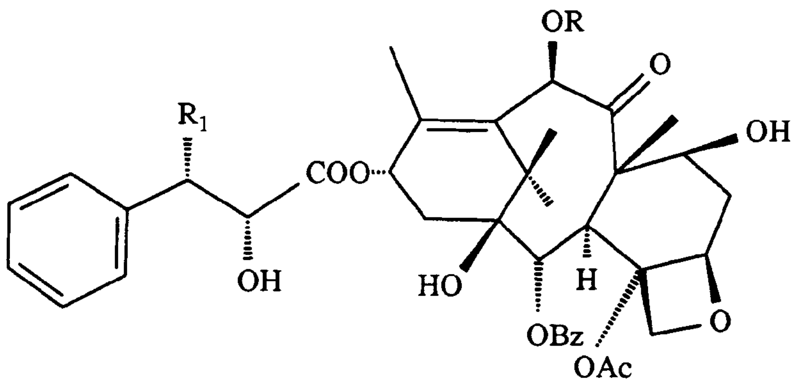

(I)

dans laquelle R représente un atome d'hydrogène ou un radical acétyle, le symbole $R_1$ représente un radical tertiobutoxycarbonylamino ou benzoyloxyamino en solution dans un mélange d'éthanol de polysorbate.

**-2-** Compositions selon la revendication 1 caractérisées en ce que dans le composé de formule (I) R représente un groupe hydrogène et $R_1$ un radical tertiobutoxycarbonylamino.

**-3-** Compositions selon la revendication 1 caractérisées en ce que dans le composé de formule (I) R représente un groupe acétyle et $R_1$ représente un radical benzoyloxyamino.

**-4-** Compositions selon l'une quelconque des revendications précédentes caractérisées en ce qu'elles contiennent entre 6 et 15 mg/ml de composé de formule (I).

**-5-** Perfusion caractérisée en ce qu'elle contient environ 1 mg/ml ou moins de composé de formule (I) et qu'elle contient moins de 35 ml/l d'éthanol et moins de 35 ml/l d'agent tensioactif.

Office européen des brevets

# RAPPORT DE RECHERCHE EUROPEENNE

Numero de la demande

EP 92 40 1903

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,Y | JOURNAL OF THE NATIONAL CANCER INSTITUTE, vol. 82, no. 15, 1 août 1990, pages 1247-1259; E.K. ROWINSKY et al.: "Taxol: a novel investigational antimicrotubule agent"<br>* Page 1251, colonne de gauche: "Pharmaceutical data" *<br>--- | 1-5 | A 61 K 47/10<br>A 61 K 47/26<br>A 61 K 47/44 |
| Y | EP-A-0 253 738 (RHONE-POULENC SANTE)<br>* Revendications 1,4; page 8, lignes 1-16; page 8, exemple *<br>--- | 1-5 | |
| Y | STN INTERNATIONAL INFORMATION SERVICES, BASE DE DONNEES: CHEMICAL ABSTRACTS, vol. 106, no. 22, 1987, abrégé no. 182581c, Columbus, Ohio, US; B.D. TARR et al.: "A new parenteral vehicle for the administration of some poorly water soluble anti-cancer drugs", & J. PARENTER. SCI. TECHNOL., 41(1), 31-3<br>* Abrégé *<br>--- | 1-5 | **DOMAINES TECHNIQUES RECHERCHES (Int. Cl.5)** |
| A | EP-A-0 118 316 (LIPID SPECIALITIES)<br>* Revendications 1,19,21; page 14, exemple 10 *<br>----- | 1-5 | A 61 K |

Le présent rapport a été établi pour toutes les revendications

| Lieu de la recherche | Date d'achèvement de la recherche | Examinateur |
|---|---|---|
| LA HAYE | 21-09-1992 | SCARPONI U. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)